(19)

Europäisches
Patentamt
European
Patent Office
Office européen
des brevets

(11) **EP 2 415 496 A1**

(12) **EUROPEAN PATENT APPLICATION**
published in accordance with Art. 153(4) EPC

(43) Date of publication:
**08.02.2012 Bulletin 2012/06**

(21) Application number: **10758686.9**

(22) Date of filing: **30.03.2010**

(51) Int Cl.:
*A61M 25/01* (2006.01)          *A61B 18/12* (2006.01)

(86) International application number:
**PCT/JP2010/055633**

(87) International publication number:
**WO 2010/113915 (07.10.2010 Gazette 2010/40)**

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **31.03.2009 JP 2009087189**

(71) Applicant: **Toray Industries, Inc.
Tokyo, 103-8666 (JP)**

(72) Inventors:
• **MATSUKUMA, Akinori
Otsu-shi
Shiga 520-2141 (JP)**

• **TAKAOKA, Motoki
Otsu-shi
Shiga 520-2141 (JP)**
• **YAGI, Takahiro
Otsu-shi
Shiga 520-2141 (JP)**

(74) Representative: **Kador & Partner
Corneliusstrasse 15
80469 München (DE)**

(54) **GUIDE WIRE AND BALLOON-EQUIPPED ABLATION CATHETER SYSTEM WITH SAME**

(57)    A balloon-equipped ablation catheter used to efficiently cauterize a cardiac wall. A guide wire for a balloon-equipped ablation catheter, provided with a bending section, wherein the bending section has a bending region provided in that region of the guide wire which has a length of at least 20 mm in the longitudinal direction of the guide wire and having bending rigidity in the range of 570 to 3740 N·mm2, the guide wire bending angle which is the angle formed between the tangent line at the start point of the bending region and the tangent line at the end point of the bending region, said tangent lines intersecting each other, and measured in the direction of the bend from the tangent line at the start point of the bending region is in the range of 45 to 180 degrees, and the balloon bending angle which is the angle measured relative to the longitudinal direction when the guide wire is inserted in a lumen, which communicates from the distal end to the proximal end in the longitudinal direction of a shaft for the balloon-equipped ablation catheter, and the bending region is located right below the balloon is in the range of 30 to 90 degrees.

Fig. 1

EP 2 415 496 A1

**Description**

TECHNICAL FIELD

The present invention relates to a guidewire and an ablation catheter with a balloon having the same.

BACKGROUND ART

An ablation catheter with a balloon is a medical device to be used for treatment of cardiac arrhythmias such as a paroxysmal supraventricular tachycardia, an atrial tachycardia, an atrial flutter, and a paroxysmal ventricular tachycardia.
An electric isolation of a pulmonary vein with use of the ablation catheter with a balloon (pulmonary venous opening ablation) is conducted by introducing a balloon attached to a front portion of the catheter into an inferior vena cava percutaneously, making the balloon reach a left atrium via an atrial septum from a right atrium of a heart, expanding the balloon, heating a surface of the balloon by radio-frequency power, and heating an annular periphery of a pulmonary venous opening (Patent Literatures 1 and 2).
In the treatment with use of the ablation catheter with a balloon, a guidewire is used in some cases to guide the balloon into the pulmonary venous opening and bring the balloon into close contact with the pulmonary venous opening. Normally, the guidewire has a linear main body portion and a flexible front portion, and the front portion is elaborated to be formed in a J shape in order to prevent the front portion from damaging a vascularized tissue.
Also, in the treatment of the cardiac arrhythmia, not only the pulmonary venous opening but also a part between a mitral annulus and the pulmonary venous opening, a part between a left pulmonary venous opening and a left atrial appendage, and a cardiac wall between pulmonary venous openings need to be ablated to block the paths of various electric signals that cause the arrhythmia. In such ablation of the cardiac wall, the ablation catheter with a balloon is not used, but an ablation catheter having a metal chip with a length of 4 mm at a front end is used.

PRIOR ART REFERENCES

PATENT LITERATURE

Patent Literature 1: Japanese Patent Application Laid-Open No. 2002-78809
Patent Literature 2: Japanese Patent No. 4062935

SUMMARY OF THE INVENTION

PROBLEMS TO BE SOLVED BY THE INVENTION

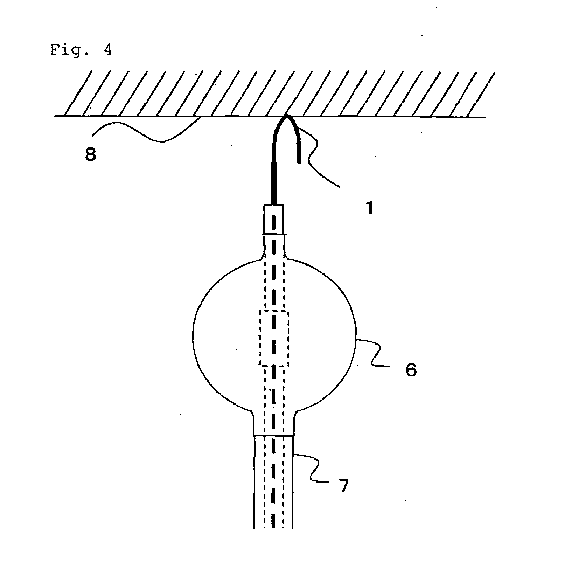

However, in the treatment with use of the ablation catheter having the metal chip at the front end, a tissue area that can be ablated at a single ablation is small, and an ablation line cannot be formed efficiently in many cases, which causes a failure in formation of the ablation line in the treatment of the cardiac arrhythmia.
On the other hand, the ablation catheter with a balloon is **characterized in that** the tissue area that can be ablated at a single ablation is large but can hardly be used in the cardiac wall ablation since the balloon cannot be brought into close contact with the cardiac wall structurally as a guidewire 1 and a balloon front portion are obstacles as shown in Fig. 4.
It is an object of the present invention to enable efficient ablation of a cardiac wall with use of an ablation catheter with a balloon.

SOLUTION TO THE PROBLEMS

As a result of concerted study directed toward solving the aforementioned problem, the present inventors discovered the following inventions (1) to (3).

(1) A guidewire for an ablation catheter with a balloon having a curved portion, wherein the curved portion has a curved region having bending stiffness in a range of 570 to 3740 N·mm$^2$ in a region of the guidewire having a length of at least 20 mm in a longitudinal direction of the guidewire, a guidewire curving angle formed in a curving direction from a tangent line at a start point of the curved region by intersecting the tangent line at the start point of the curved region with a tangent line at an end point of the curved region is in a range of 45 to 180 degrees, and when the guidewire is inserted into a lumen communicating from a distal end to a proximal end in a longitudinal direction of a shaft for an ablation catheter with a balloon, and the curved region is located right below a balloon, a balloon curving angle in the longitudinal direction of the balloon is in a range of 30 to 90 degrees.

(2) The guidewire according to the above (1), which is used for ablation of a cardiac wall.

(3) An ablation catheter system with a balloon comprising the guidewire according to the above (1) or (2).

(4) A treatment method of a cardiac arrhythmia using the ablation catheter system with a balloon according to the above (3).

EFFECTS OF THE INVENTION

With the present invention, a balloon of an ablation catheter with a balloon can be brought into close contact with a cardiac wall, and efficient ablation of the cardiac wall can be achieved. Also, with the present invention, ablation of the cardiac wall can be done by the ablation catheter with a balloon, and a wide range of a tissue can be ablated at a single ablation, which can eliminate a failure in formation of an ablation line, which occurs at the time of treatment of a cardiac arrhythmia with use of an ablation catheter having a metal chip at a front end.

BRIEF DESCRIPTION OF THE DRAWINGS

Fig. 1 is a schematic view showing a guidewire for an ablation catheter with a balloon according to a first embodiment of the present invention.

Fig. 2A is a schematic view showing a state of applying a balloon to a cardiac wall at a shallow angle in an ablation catheter system with a balloon having the guidewire for the ablation catheter with a balloon according to the first embodiment of the present invention.

Fig. 2B is a schematic view showing a state of applying the balloon to the cardiac wall at a deep angle in the ablation catheter system with a balloon having the guidewire for the ablation catheter with a balloon according to the first embodiment of the present invention.

Fig. 3 is a schematic view showing an embodiment of the ablation catheter system with a balloon having the guidewire for the ablation catheter with a balloon according to the first embodiment of the present invention.

Fig. 4 is a schematic view showing a cardiac wall ablation by an ablation catheter system with a balloon according to the related art.

DESCRIPTION OF PREFERRED EMBODIMENTS

Preferred embodiments of the present invention will be described below with reference to the accompanying drawings, but the present invention is not limited to these embodiments. Like reference characters designate similar or identical parts throughout the several views thereof, and duplicate explanation is omitted. Also, the ratio in the drawings does not necessarily correspond to an actual ratio.

A guidewire for an ablation catheter with a balloon according to the present invention is a guidewire having a curved portion, wherein the curved portion has a curved region having bending stiffness in a range of 570 to 3740 N·mm$^2$ in a region of the guidewire having a length of at least 20 mm in a longitudinal direction of the guidewire, a guidewire curving angle formed in a curving direction from a tangent line at a start point of the curved region by intersecting the tangent line at the start point of the curved region with a tangent line at an end point of the curved region is in a range of 45 to 180 degrees, and when the guidewire is inserted into a lumen communicating from a distal end to a proximal end in a longitudinal direction of a shaft for an ablation catheter with a balloon, and the curved region is located right below a balloon, a balloon curving angle in the longitudinal direction of the balloon is in a range of 30 to 90 degrees.

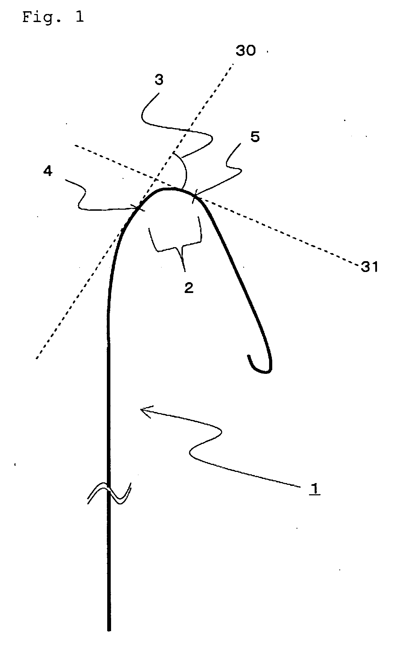

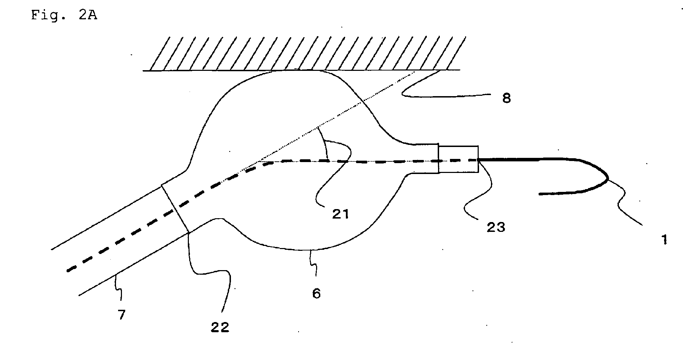

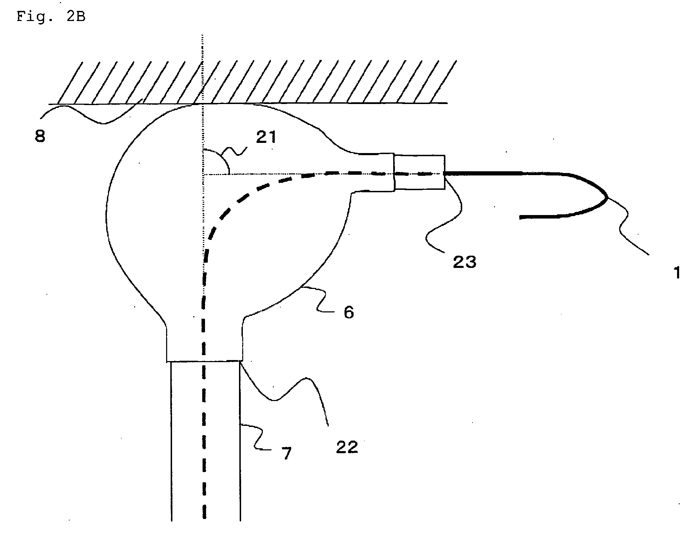

Fig. 1 is a schematic view showing a guidewire 1 for an ablation catheter with a balloon according to a first embodiment of the present invention. Also, Fig. 2A is a schematic view showing a state of applying a balloon to a cardiac wall at a shallow angle in an ablation catheter system with a balloon having the guidewire for the ablation catheter with a balloon according to the first embodiment of the present invention, and Fig. 2B is a schematic view showing a state of applying the balloon to the cardiac wall at a deep angle in the ablation catheter system with a balloon having the guidewire for the ablation catheter with a balloon according to the first embodiment of the present invention.

"The bending stiffness" is a product of Young's modulus E of a material for the guidewire and second moment of area I as calculated by Equation 1 shown below and is preferably in a range of 570 to 3740 N·mm$^2$.

```
Bending stiffness =

Young's modulus E × second moment of area I  ··· Equation 1
```

As for Young's modulus E, approximation in Equation 2 shown below is established when a material for the guidewire is SUS304WPB, for example.
$$\text{Young's modulus } E \approx 186 \times 10^3 \text{N/mm}^2 \quad \cdots \text{ Equation 2}$$

As for second moment of area I, approximation in Equation 3 shown below is established when a cross-sectional shape of the guidewire is a circle, for example.
$$\text{second moment of area } I \approx \pi \times (\text{radius})^4/4$$
$$\cdots \text{ Equation 3}$$

Examples of a material for the guidewire 1 include a metal such as stainless steel and an alloy.
The length of the guidewire 1 is preferably 0.5 to 2.5 m from a viewpoint of practicality.
The length of the curved region is preferably 20 to 25 mm from a viewpoint of adapting to 20 to 40 mm, which is a general diameter of a balloon 6.
"The guidewire curving angle" is an angle formed in the curving direction from the tangent line at the start point of the curved region by intersecting the tangent line at the start point of the curved region with the tangent line at the end point of the curved region and is shown as a guidewire curving angle 3 in Fig. 1, for example.
The start point of the curved region refers to a certain point in the curved portion of the guidewire and is shown as a start point 4 of the curved region in Fig. 1, for example. The tangent line at the start point of the curved region is shown as a tangent line 30 at the start point 4 of the curved region in Fig. 1, for example.
The end point of the curved region refers to an arbitrary point in the curved portion of the guidewire arbitrarily selected so that the curved region may have a length of at least 20 mm from the start point of the curved region. It is shown as an end point 5 of the curved region in Fig. 1. The tangent line at the end point of the curved region is shown as a tangent line 31 at the end point 5 of the curved region in Fig. 1, for example.
"The curved region" is a specific region of the guidewire between the start point of the curved region and the arbitrary end point of the curved region in the curved portion of the guidewire and is shown as a curved region 2 in Fig. 1, for example.
The guidewire for the ablation catheter with a balloon according to the present invention includes any guidewire that is shaped to have, in the curved portion of the guidewire, one or more curved regions having a length of at least 20 mm and having bending stiffness in a range of 570 to 3740 N·mm$^2$.
"The balloon curving angle" is an angle formed by a tangent line of the catheter shaft and a tangent line of the guidewire at a catheter front end and is shown as an angle formed by a tangent line of a catheter shaft 7 and a tangent line of the guidewire 1 at a catheter front end 23, that is, a balloon curving angle 21, in Fig. 2A or 2B, for example.
The balloon curving angle 21 is preferably 30 to 90 degrees and is more preferably 90 degrees from a viewpoint of bringing the balloon 6 into close contact with a cardiac wall 8.
A front end of the guidewire 1 is preferably flexible in consideration of possibility of damage of a myocardial tissue in a case of contacting the tissue and is more preferably a J shape shown in Fig. 1.
The guidewire for the ablation catheter with a balloon according to the present invention is preferably used for ablation of the cardiac wall from a viewpoint of enabling the balloon of the ablation catheter with a balloon to closely contact the cardiac wall.
Also, the ablation catheter system with a balloon according to the present invention includes the guidewire for the ablation catheter with a balloon according to the present invention.

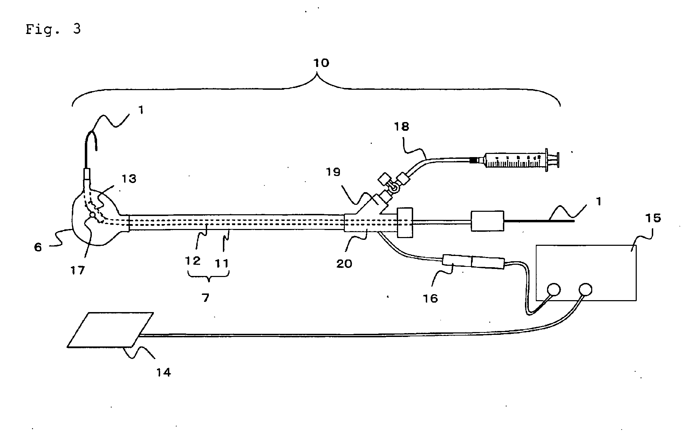

Fig. 3 is a schematic view showing an embodiment of the ablation catheter system with a balloon having the guidewire for the ablation catheter with a balloon according to the first embodiment of the present invention.
The ablation catheter system with a balloon shown in Fig. 3 has on a front side of an ablation catheter with a balloon 10 the balloon 6 that can expand and contract and has the double-cylinder catheter shaft 7 in which an inner side tube body 12 is inserted in a lumen of an outer side tube body 11. A front portion of the balloon 6 is fixed to a front portion in a longitudinal direction of the inner side tube body 12 while a rear portion of the balloon 6 is fixed to a front portion in a longitudinal direction of the outer side tube body 11.

The length of each of the outer side tube body 11 and the inner side tube body 12 is preferably 0.5 to 2 m and is more preferably 0.8 to 1.2 m.

A material for each of the outer side tube body 11 and the inner side tube body 12 is preferably a flexible material with excellent antithrombogenicity such as a fluorine resin, a polyamide resin, a polyurethane resin, or a polyimide resin.

Bending stiffness of the catheter having a lumen allowing the guidewire 1 to pass therethrough (e.g., the inner side tube body 11) is preferably higher than bending stiffness of the guidewire 1. Keeping the bending stiffness of the catheter higher than the bending stiffness of the guidewire 1 can avoid deformation of the catheter itself when the guidewire 1 is inserted.

The shape of the balloon 6 may be any shape as long as it can fit in a pulmonary venous opening, and examples of the shape include a spherical shape having a diameter of 20 to 40 mm and a tapered conical outer shape.

The film thickness of the balloon 6 is preferably 20 to 200 $\mu$m and is more preferably 30 to 100 $\mu$m.

A material for the balloon 6 is preferably a stretchable material with excellent antithrombogenicity and is more preferably a polyurethane polymeric material.

Examples of the polyurethane polymeric material include thermoplastic polyether urethane, polyether polyurethane urea, fluorine polyether urethane urea, a polyether polyurethane urea resin, and polyether polyurethane urea amide.

A radio-frequency carrying electrode 13 is arranged in an interior of the balloon 6.

In a case where the radio-frequency carrying electrode 13 is to be fixed to the inner side tube body 12, examples of a fixing method include caulking, adhesive, welding, and a heat shrinkable tube, but the radio-frequency carrying electrode 13 does not have to be fixed to the inner side tube body 12.

The balloon is heated by supplying radio-frequency power between the radio-frequency carrying electrode 13 and an off-balloon electrode 14 attached to a surface of a patient's body by a radio-frequency generator 15, and plural radio-frequency carrying electrodes 13 may be arranged in the interior of the balloon 6 to supply the radio-frequency power among the radio-frequency carrying electrodes. Also, from a viewpoint of improving flexibility of the balloon in a range in which the radio-frequency carrying electrode 13 has been arranged, the radio-frequency carrying electrode 13 may be divided into plural pieces and arranged.

The shape of the radio-frequency carrying electrode 13 is not particularly limited and is preferably a tubular shape such as a coiled shape or a cylindrical shape.

The diameter of an electric wire of the coiled radio-frequency carrying electrode 13 is preferably 0.1 to 1 mm and is more preferably 0.2 to 0.5 mm from a viewpoint of practicality.

A material for the radio-frequency carrying electrode 13 is preferably a highly conductive metal.

Examples of the highly conductive metal include highly conductive metals such as silver, gold, platinum, and copper.

A radio-frequency power supplying lead wire connected to the radio-frequency carrying electrode 13 is connected to the radio-frequency generator 15 via an electrode connector 16 and transmits radio-frequency currents to the radio-frequency carrying electrode 13.

The radio-frequency power supplying lead wire is connected to the radio-frequency carrying electrode 5 by soldering, caulking, or the like.

The diameter of the radio-frequency power supplying lead wire is preferably 0.1 to 1 mm and is more preferably 0.2 to 0.5 mm from a viewpoint of practicality.

Examples of a material for the radio-frequency power supplying lead wire include highly conductive metals such as copper, silver, gold, platinum, tungsten, and an alloy, the radio-frequency power supplying lead wire is preferably provided with an electrical insulating protective coat such as a fluorine resin from a viewpoint of preventing short circuit, and it is more preferable to form a part of the radio-frequency power supplying lead wire, from which the electrical insulating protective coat has been stripped away, in a coiled shape and use the part as the radio-frequency carrying electrode 13 from a viewpoint of dispensing with connection by soldering, caulking, or the like.

A temperature sensor 17 is fixed to the inner side tube body 12, the radio-frequency carrying electrode 13, or an inner surface of the balloon 6. Plural temperature sensors 17 may be fixed from a viewpoint of backup in a case of a failure of the temperature sensor.

Examples of the temperature sensor 17 include a thermocouple and a resistance-temperature detector.

A temperature sensor lead wire connected to the temperature sensor 17 is connected to the radio-frequency generator 15 via the electrode connector 16 and transmits a temperature signal measured at the temperature sensor 17 to the radio-frequency generator 15.

When the temperature sensor 17 is a thermocouple, a material for the temperature sensor lead wire is preferably the same material as that for the thermocouple, and examples of the material include copper and constantan when the temperature sensor 17 is a T-type thermocouple. On the other hand, when the temperature sensor 17 is a resistance-temperature detector, a material for the temperature sensor lead wire is preferably a highly conductive metal such as copper, silver, gold, platinum, tungsten, or an alloy. Meanwhile, the temperature sensor lead wire is preferably provided with an electrical insulating protective coat such as a fluorine resin from a viewpoint of preventing short circuit.

Also, the ablation catheter with a balloon 10 shown in Fig. 3 has a tube connecting portion 19 having a through hole to which a balloon expanding/contracting tube 18 for supplying a liquid to the interior of the balloon 6 is attached. The tube connecting portion 19 communicates with a space between the outer side tube body 11 and the inner side tube body 12.

The tube connecting portion 19 is preferably provided at the outer side tube body, a stopcock, a lid, or a coupling member arranged on a rear side in the longitudinal direction of the outer side tube body, and the tube connecting portion 19 of the ablation catheter with a balloon 10 shown in Fig. 3 is provided at a coupling member 20.

The radio-frequency power supplying lead wire and the temperature sensor lead wire are preferably inserted into the space between the outer side tube body 11 and the inner side tube body 12 from the radio-frequency carrying electrode 13 and the temperature sensor 17 and arranged so as to be taken outside from the coupling member 20.

The guidewire 1 for the ablation catheter with a balloon is inserted into a lumen of the inner side tube body 12.

EXAMPLES

Hereinafter, specific examples of the guidewire and the ablation catheter system with a balloon having the same according to the present invention will be described with reference to the drawings.

(EXAMPLE 1)

The ablation catheter system with a balloon according to a first embodiment of the present invention was prepared as follows.

A stainless steel wire (SUS304WPB wire) having a cross-sectional shape of a circle with a diameter of 0.5 mm and having a length of 2000 mm was prepared as a guidewire, and with a position 1950 mm distanced in length from its one end set as the start point 4 of a curved region, the curved region 2 was formed so that the entire curved portion might be a curved region having a length of 20 mm (hereinafter referred to as EXAMPLE 1 guidewire).

Bending stiffness of EXAMPLE 1 guidewire is 570 N·mm$^2$ when Young's modulus E is $186 \times 10^3$N/mm$^2$, and when second moment of area I is $\pi \times (0.25)^4/4$.

Subsequently, the ablation catheter with a balloon according to a first embodiment of the present invention was prepared as follows.

First, the balloon 6 having a diameter of 30 mm and a thickness of 50 $\mu$m was prepared by dipping, in which a balloon mold made of glass having a mold surface corresponding to a desired balloon shape was immersed into a polyurethane solution having a concentration of 13% and was heated to evaporate a solvent to form an urethane polymer film on the surface of the mold.

The coupling member 20 provided with the tube connecting portion 19 was inserted and fitted in a rear end of the outer side tube body 11, which was a tube made of polyurethane having an outer diameter of 4 mm, an inner diameter of 3 mm, and an entire length of 1000 mm, and was attached and fixed.

With a position 20 mm distanced from a front end of the inner side tube body 12, which was a tube made of polyurethane having an outer diameter of 1.7 mm, an inner diameter of 1.2 mm, and an entire length of 1100 mm, set as a starting point, after a part of the electrical insulating protective coat provided on the radio-frequency power supplying lead wire, which was an electric soft copper wire plated with silver having a diameter of 0.5 mm, was stripped away, the radio-frequency power supplying lead wire was directly wound around the inner side tube body 12 to form a coiled shape having a length of 10 mm and use it as the radio-frequency carrying electrode 13.

An extra fine thermocouple copper wire provided with the electrical insulating protective coat as one temperature sensor lead wire and an extra fine thermocouple constantan wire provided with the electrical insulating protective coat as the other temperature sensor lead wire were connected at the tip ends and were reinforced by soldering, and the connected portion was used as the temperature sensor 17. The temperature sensor 17 was fixed at a position 3 mm distanced from a front end of the radio-frequency carrying electrode 13 by caulking.

The inner side tube body 12 to which the radio-frequency carrying electrode 13 and the temperature sensor 17 were fixed was inserted into the outer side tube body 11 from a rear side of the coupling member 20 and was fixed to the coupling member 20 by a lid.

The radio-frequency power supplying lead wire and the temperature sensor lead wire were inserted into the space between the outer side tube body 11 and the inner side tube body 12 from the radio-frequency carrying electrode 13 and the temperature sensor 17, were taken outside from the coupling member 20, and were connected to the electrode connector 16.

The front portion of the balloon 6 was fixed on an outer circumference of the inner side tube body 12 at a position 10 mm distanced from the front end of the inner side tube body 12 by thermal welding while the rear portion of the balloon 6 was thermally welded on an outer circumference of a front portion of the outer side tube body 11 to prepare the ablation catheter with a balloon according to the first embodiment of the present invention.

Finally, the balloon 6 was supplied with saline and was expanded so that the maximum diameter thereof might be 20 mm, EXAMPLE 1 guidewire was then inserted into the lumen of the inner side tube body 12 of the ablation catheter with a balloon according to the first embodiment of the present invention, and the ablation catheter system with a balloon according to the first embodiment of the present invention (hereinafter referred to as EXAMPLE 1 catheter system) was completed.

(EXAMPLE 2)

The ablation catheter system with a balloon according to a second embodiment of the present invention was prepared as follows.

A stainless steel wire (SUS304WPB wire) having a cross-sectional shape of a circle with a diameter of 0.8 mm and having a length of 2000 mm was prepared as a guidewire, and with a position 1950 mm distanced in length from its one end set as the start point 4 of a curved region, the curved region 2 was formed so that the entire curved portion might be a curved region having a length of 20 mm (hereinafter referred to as EXAMPLE 2 guidewire).

Bending stiffness of EXAMPLE 2 guidewire is 3740 N· mm$^2$ when Young's modulus E is $186 \times 10^3$N/mm$^2$, and when second moment of area I is $\pi \times (0.4)^4/4$.

Subsequently, the balloon 6 of the same ablation catheter with a balloon as that prepared in EXAMPLE 1 was supplied with saline and was expanded so that the maximum diameter thereof might be 20 mm, EXAMPLE 2 guidewire was then inserted into the lumen of the inner side tube body 12, and the ablation catheter system with a balloon according to the second embodiment of the present invention (hereinafter referred to as EXAMPLE 2 catheter system) was completed.

(COMPARATIVE EXAMPLE 1)

As COMPARATIVE EXAMPLE 1, the ablation catheter system with a balloon was prepared as follows.

A stainless steel wire (SUS304WPB wire) having a cross-sectional shape of a circle with a diameter of 0.4 mm and having a length of 2000 mm was prepared as a guidewire, and with a position 1950 mm distanced in length from its one end set as a start point of a curved region, the curved region was formed so that the entire curved portion might be a curved region having a length of 20 mm (hereinafter referred to as COMPARATIVE EXAMPLE 1 guidewire).

Bending stiffness of COMPARATIVE EXAMPLE 1 guidewire is 230 N·mm$^2$ when Young's modulus E is $186 \times 10^3$N/mm$^2$, and when second moment of area I is $\pi \times (0.25)^4/4$.

Subsequently, the balloon 6 of the same ablation catheter with a balloon as that prepared in EXAMPLE 1 was supplied with saline and was expanded so that the maximum diameter thereof might be 20 mm, COMPARATIVE EXAMPLE 1 guidewire was then inserted into the lumen of the inner side tube body, and the ablation catheter system with a balloon (hereinafter referred to as COMPARATIVE EXAMPLE 1 catheter system) was completed.

(COMPARATIVE EXAMPLE 2)

As COMPARATIVE EXAMPLE 2, the ablation catheter system with a balloon was prepared as follows.

A stainless steel wire (SUS304WPB wire) having a cross-sectional shape of a circle with a diameter of 0.84 mm and having a length of 2000 mm was prepared as a guidewire, and with a position 1950 mm distanced in length from its one end set as a start point of a curved region, the curved region was formed so that the entire curved portion might be a curved region having a length of 20 mm (hereinafter referred to as COMPARATIVE EXAMPLE 2 guidewire).

Bending stiffness of COMPARATIVE EXAMPLE 2 guidewire is 4550 N·mm$^2$ when Young's modulus E is $186 \times 10^3$N/mm$^2$, and when second moment of area I is $\pi \times (0.42)^4/4$.

Subsequently, the balloon 6 of the same ablation catheter with a balloon as that prepared in EXAMPLE 1 was supplied with saline and was expanded so that the maximum diameter thereof might be 20 mm, COMPARATIVE EXAMPLE 2 guidewire was then inserted into the lumen of the inner side tube body, and the ablation catheter system with a balloon (hereinafter referred to as COMPARATIVE EXAMPLE 2 catheter system) was completed.

(COMPARATIVE EXAMPLE 3)

As COMPARATIVE EXAMPLE 3, the ablation catheter system with a balloon was prepared as follows.

A stainless steel wire (SUS304WPB wire) having a cross-sectional shape of a circle with a diameter of 1.0 mm and having a length of 2000 mm was prepared as a guidewire, and with a position 1950 mm distanced in length from its one end set as the start point 4 of a curved region, the curved region 2 was formed so that the entire curved portion might be a curved region having a length of 20 mm (hereinafter referred to as COMPARATIVE EXAMPLE 3 guidewire).

Bending stiffness of COMPARATIVE EXAMPLE 3 guidewire is 9130 N·mm$^2$ when Young's modulus E is 186 $\times$ 10$^3$N/mm$^2$, and when second moment of area I is $\pi \times (0.5)^4/4$.

Subsequently, the balloon 6 of the same ablation catheter with a balloon as that prepared in EXAMPLE 1 was supplied with saline and was expanded so that the maximum diameter thereof might be 20 mm, COMPARATIVE EXAMPLE 3 guidewire was then inserted into the lumen of the inner side tube body, and the ablation catheter system with a balloon (hereinafter referred to as COMPARATIVE EXAMPLE 3 catheter system) was completed.

(Examination of Ablation Catheter Systems with Balloon)

In each of EXAMPLE 1 catheter system, EXAMPLE 2 catheter system, COMPARATIVE EXAMPLE 1 catheter system, COMPARATIVE EXAMPLE 2 catheter system, and COMPARATIVE EXAMPLE 3 catheter system, the guidewire curving angle required for the balloon curving angle to be in a range of 30 to 90 degrees was examined. The result is shown in Table 1.

[Table 1]

| Balloon curving angle | 30 degrees | 90 degrees |
|---|---|---|
| EXAMPLE 1 guidewire curving angle (Bending stiffness: 570 N·mm$^2$) | 90 degrees | 180 degrees |
| EXAMPLE 2 guidewire curving angle (Bending stiffness: 3740 N·mm$^2$) | 45 degrees | 120 degrees |
| COMPARATIVE EXAMPLE 1 guidewire curving angle (Bending stiffness: 230 N·mm$^2$) | 180 degrees | Not practiced |
| COMPARATIVE EXAMPLE 2 guidewire curving angle (Bending stiffness: 4550 N·mm$^2$) | 45 degrees | 120 degrees |
| COMPARATIVE EXAMPLE 3 guidewire curving angle (Bending stiffness: 9130 N·mm$^2$) | 40 degrees | 100 degrees |

In COMPARATIVE EXAMPLE 1 catheter system, the guidewire curving angle needs to be 180 degrees or more, and a case of exceeding 180 degrees will cause unfavorable operability of the guidewire at the time of insertion into the catheter, which is not favorable. Also, even in a case of approximately 360 degrees, the balloon curving angle was not 90 degrees, and the catheter system was determined not to be applicable.

In COMPARATIVE EXAMPLE 2 catheter system, the balloon curving angle was 90 degrees in a case where the guidewire curving angle was 120 degrees, but resistance occurred when COMPARATIVE EXAMPLE 2 guidewire was inserted into the lumen of the inner side tube body 12 of the ablation catheter with a balloon, and thus the catheter system was determined not to be applicable.

In COMPARATIVE EXAMPLE 3 catheter system, the balloon curving angle was 90 degrees in a case where the guidewire curving angle was 100 degrees, but significant resistance occurred to make insertion difficult when COMPARATIVE EXAMPLE 3 guidewire was inserted into the lumen of the inner side tube body 12 of the ablation catheter with a balloon, and thus the catheter system was determined not to be applicable.

As is apparent from the result of the above examination, optimal bending stiffness of the guidewire for the ablation catheter system with a balloon according to the present invention is in a range of 570 to 3740 N·mm$^2$, and an optimal guidewire curving angle is in a range of 45 to 180 degrees.

With the ablation catheter system with a balloon according to the present invention, the balloon of the ablation catheter with a balloon can be curved to have an angle applicable to ablation of the cardiac wall by the curved region on the front side in the longitudinal direction of the guidewire for the ablation catheter with a balloon, and it is apparent that the ablation catheter system with a balloon in which a conventional ablation catheter with a balloon is applicable to the cardiac wall ablation can be provided.

INDUSTRIAL APPLICABILITY

The present invention can be used as an ablation catheter system with a balloon that ablates a cardiac wall.

DESCRIPTION OF REFERENCE SIGNS

1 ..: guidewire for an ablation catheter with a balloon, 2 ... curved region, 3 ... guidewire curving angle, 4 ... start point of a curved region, 5 ... end point of a curved region, 6 ... balloon, 7 ... double-cylinder catheter shaft, 8 ... cardiac wall, 10 ... ablation catheter with a balloon, 11 ... outer side tube body, 12 ... inner side tube body, 13 ... radio-frequency carrying electrode, 14 ... off-balloon electrode, 15 ... radio-frequency generator, 16 ... electrode connector, 17 ... temperature sensor, 18 ... balloon expanding/contracting tube, 19 ... tube connecting portion, 20 ... coupling member, 21 ... balloon curving angle, 22 ... balloon jointing portion, 23 ... catheter front end, 30 ... tangent line at a start point of a curved region, 31 ... tangent line at an end point of a curved region

**Claims**

1.  A guidewire for an ablation catheter with a balloon having a curved portion,
    wherein the curved portion has a curved region having bending stiffness in a range of 570 to 3740 N·mm$^2$ in a region of the guidewire having a length of at least 20 mm in a longitudinal direction of the guidewire,
    a guidewire curving angle formed in a curving direction from a tangent line at a start point of the curved region by intersecting the tangent line at the start point of the curved region with a tangent line at an end point of the curved region is in a range of 45 to 180 degrees, and
    when the guidewire is inserted into a lumen communicating from a distal end to a proximal end in a longitudinal direction of a shaft for an ablation catheter with a balloon, and the curved region is located right below a balloon, a balloon curving angle in the longitudinal direction of the balloon is in a range of 30 to 90 degrees.

2.  The guidewire according to claim 1, which is used for ablation of a cardiac wall.

3.  An ablation catheter system with a balloon comprising the guidewire according to claim 1 or 2.

4.  A treatment method of a cardiac arrhythmia using the ablation catheter system with a balloon according to claim 3.

Fig. 1

Fig. 2A

Fig. 2B

Fig. 3

Fig. 4

EP 2 415 496 A1

## INTERNATIONAL SEARCH REPORT

| International application No. |
|---|
| PCT/JP2010/055633 |

**A. CLASSIFICATION OF SUBJECT MATTER**
*A61M25/01*(2006.01)i, *A61B18/12*(2006.01)i

According to International Patent Classification (IPC) or to both national classification and IPC

**B. FIELDS SEARCHED**

Minimum documentation searched (classification system followed by classification symbols)
A61M25/01, A61B18/12

Documentation searched other than minimum documentation to the extent that such documents are included in the fields searched
Jitsuyo Shinan Koho          1922-1996    Jitsuyo Shinan Toroku Koho   1996-2010
Kokai Jitsuyo Shinan Koho   1971-2010    Toroku Jitsuyo Shinan Koho   1994-2010

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2002-523152 A  (Cook,Inc.),<br>30 July 2002 (30.07.2002),<br>paragraphs [0011] to [0018]; fig. 1, 5<br>& US 6254550 B1        & WO 2000/010636 A1 | 1-3 |
| Y | JP 2002-78809 A  (Shutaro SATAKE),<br>19 March 2002 (19.03.2002),<br>paragraphs [0021] to [0031]; fig. 1<br>& US 2002/0029062 A1 | 1-3 |
| Y | JP 2003-508168 A  (Schneider (Europe) GmbH),<br>04 March 2003 (04.03.2003),<br>paragraphs [0019] to [0025]; fig. 7<br>& US 7048695 B1        & WO 2001/017601 A1 | 1-3 |

☐ Further documents are listed in the continuation of Box C.     ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 07 May, 2010 (07.05.10) | 18 May, 2010 (18.05.10) |

| Name and mailing address of the ISA/<br>    Japanese Patent Office | Authorized officer |
|---|---|
| Facsimile No. | Telephone No. |

Form PCT/ISA/210 (second sheet) (July 2009)

| **INTERNATIONAL SEARCH REPORT** | International application No. |
|---|---|
| | PCT/JP2010/055633 |

**Box No. II      Observations where certain claims were found unsearchable (Continuation of item 2 of first sheet)**

This international search report has not been established in respect of certain claims under Article 17(2)(a) for the following reasons:

1. [×] Claims Nos.: 4
   because they relate to subject matter not required to be searched by this Authority, namely:
   Claim 4 pertains to a method for treatment of the human body by therapy and thus relates to a subject matter on which the International Searching Authority is not required to carry out a search under the provisions of PCT Article 17(2)(a)(i) and PCT Rule 39.1(iv).

2. [ ] Claims Nos.:
   because they relate to parts of the international application that do not comply with the prescribed requirements to such an extent that no meaningful international search can be carried out, specifically:

3. [ ] Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying additional fees, this Authority did not invite payment of additional fees.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**      [ ] The additional search fees were accompanied by the applicant's protest and, where applicable, the payment of a protest fee.

     [ ] The additional search fees were accompanied by the applicant's protest but the applicable protest fee was not paid within the time limit specified in the invitation.

     [ ] No protest accompanied the payment of additional search fees.

Form PCT/ISA/210 (continuation of first sheet (2)) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002078809 A
- JP 4062935 B